# EUROPÄISCHE PATENTSCHRIFT

(11) **EP 0 627 405 B1**
(45) Veröffentlichungstag und Bekanntmachung des Hinweises auf die Patenterteilung: **15.01.1997**
(21) Anmeldenummer: 94107082.3
(22) Anmeldetag: 06.05.1994
(51) Int. Cl.: C07C 67/307, C07C 69/94

(54) **Verfahren zur Herstellung von 5-Brom-6-methoxy-1-naphthoesäuremethylester**
Process for the preparation of methyle 5-bromo-6-methoxy-1-naphthoate
Procédé de préparation d'ester méthylique de l'acide 5-bromo-6-méthoxy-1-naphtoique

(30) Priorität: 01.06.1993 DE 4318069
(43) Veröffentlichungstag der Anmeldung: 07.12.1994
(73) Patentinhaber: HOECHST AKTIENGESELLSCHAFT, 65926 Frankfurt am Main (DE)
(72) Erfinder: Wessel, Thomas Dr., D-63477 Maintal (DE)

(56) Entgegenhaltungen:
- EP-A- 0 149 407
- EP-A- 0 200 840
- DE-A- 3 014 972
- DATABASE WPI Week 8637, Derwent Publications Ltd., London, GB; AN 86-242370 37 'META-BROMO-BENZOIC ACID PREPARATION' & JP-A-61 171 452 (MITSUBISHI CHEM IND KK) 2. August 1986

## Beschreibung

Die vorliegende Erfindung betrifft ein Verfahren zur Herstellung von 5-Brom-6-methoxy-1-naphthoesäuremethylester durch Bromierung von 6-Methoxy-1-naphthoesäuremethylester mit Brom in Gegenwart eines Oxidationsmittels.

5-Brom-6-methoxy-1-naphthoesäuremethylester ist ein wichtiges Zwischenprodukt bei der Herstellung des Pharmawirkstoffs Tolrestat, der zur Vorbeugung und Behandlung von Spätfolgen des Diabetes mellitus dient. Tolrestat hemmt das Enzym Aldosereduktase und verhindert so die bei Diabetikern verstärkte Bildung von Sorbit, die z.B. zu Nierenschäden, Nervenschäden oder Augenschäden führt (s.z.B. EP-B 059 596, EP-B 200 840, EP-A 307 519, US-A 4 808 748).

Die Herstellung von 5-Brom-6-methoxy-1-naphthoesäuremethylester(I) durch Bromierung von 6-Methoxy-1-naphthoesäuremethylester(II) mit Brom ist beschrieben.

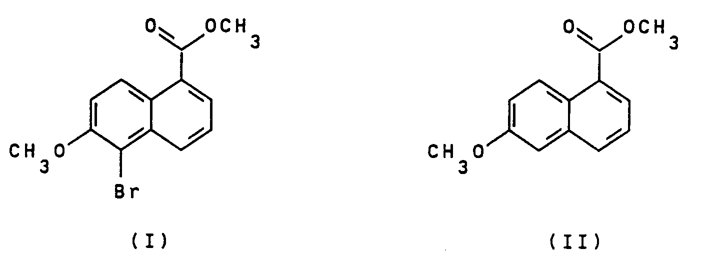

Gemäß dem Verfahren der EP-B 200 840 wird ein großer Bromüberschuß eingesetzt und die Reaktion in Gegenwart von Wasser in 1,2-Dichlorethan durchgeführt, einem halogenierten Kohlenwasserstoff, der gesundheitsschädlich und cancerogen-verdächtig ist und dessen Verwendung bei einer im technischen Maßstab erfolgenden Synthese unter ökologischen und arbeitshygienischen Aspekten bedenklich ist.

Das in der EP-B 059 596 beschriebene Bromierungsverfahren vermeidet die Verwendung von chlorierten Kohlenwasserstoffen. Als Lösungsmittel wird Essigsäure verwandt. Auch hier wird aber mit einem Brom-Überschuß (1,2 mol Br₂ pro Mol Methoxynaphthoesäureester(II) gearbeitet. Zudem ist bei dieser Verfahrensweise eine anschließende Reinigung durch Umkristallisation aus Ethanol erforderlich. Die Ausbeute beträgt nur 81 %, und der angeführte Schmelzpunkt von 119°C läßt auf eine geringe Reinheit des umkristallisierten Produkts schließen.

Bei beiden Verfahren fällt zwangsweise 1 mol Bromwasserstoff pro Mol entstandenem Brommethoxynaphthoesäureester(I) an, der im Filtrat gelöst ist oder aus der Abluft herausgewaschen werden muß und sich somit dann im Abwasser befindet, das in einem zusätzlichen Schritt einer Aufarbeitung unterzogen werden muß. Durch den Bromwasserstoffanfall und die Notwendigkeit, deutlich mehr als die äquimolare Menge Br₂ zuzugeben, wird bei den Herstellungsverfahren des Standes der Technik deutlich weniger als die Hälfte des eingesetzten Broms ausgenutzt.

Bromierungsreaktionen an Aromaten, bei denen der aus dem Brom entstehende Bromwasserstoff durch Zusatz eines Oxidationsmittels wieder in Brom überführt wird, sind bekannt. In der deutschen Patentschrift 748 621 ist ein Verfahren zur Bromierung von Phenolen und Naphtholen in Phosphoroxichlorid offenbart, bei dem durch Zugabe eines Oxidationsmittels, wie beispielsweise Schwefeltrioxid oder Oleum, eine fast quantitative Bromausnutzung erzielt wird. Von Dakka und Sasson, Journal of the Chemical Society, Chemical Communications, 1987, S.1421, ist die Bromierung von Benzol, Alkylbenzolen und Chlorbenzol mit der halben molaren Menge Br₂ unter Zusatz von wäßrigem Wasserstoffperoxid in Gegenwart eines quartären Ammoniumsalzes als Phasentransferkatalysator beschrieben. Die eingesetzten Ausgangsaromaten weisen aber keine hydrolysierbaren funktionellen Gruppen, wie Ether- und Estergruppen, auf. In dem durch den gebildeten Bromwasserstoff sauren Reaktionsmilieu ist bei Bromierungen von Ausgangsverbindungen, die solche Gruppen enthalten, mit der Bildung von Nebenprodukten durch Ester- und Etherspaltungen zu rechnen. Zur Zurückdrängung der Nebenreaktionen ist bei der Bromierung von Phenolethern und Estern aromatischer Carbonsäuren oft der Zusatz eines bromwasserstoffbindenden Mittels, wie Natriumacetat oder Calciumcarbonat, erforderlich (vgl. Houben-Weyl-Müller, Methoden der Organischen Chemie, Band V/4, S.246, S.269, S.291, Stuttgart 1960), durch den insbesondere im technischen Maßstab die Durchführung der Reaktion aufwendiger wird.

Aufgabe der vorliegenden Erfindung ist es daher, ein unter ökologischen und arbeitshygienischen Aspekten verbessertes, die Abwasserbelastung verringerndes und die Verwendung von chlorierten Kohlenwasserstoffen vermeidendes Verfahren zur Herstellung von 5-Brom-6-methoxy-1-naphthoesäuremethylester(I) bereitzustellen, das in technisch einfacher Weise in hoher Ausbeute ein hochreines Produkt liefert.

Überraschenderweise wurde gefunden, daß die Bromierung des Methoxynaphthoesäureesters(II) mit 0,5 Mol Br₂ pro Mol des Esters II oder einem unwesentlichen Überschuß in Gegenwart eines Oxidationsmittels, das in der Lage ist, Bromwasserstoff zu Brom zu oxidieren und somit den zwangsweise bei der elektrophilen aromatischen Bromierung anfallenden Bromwasserstoff zu regenerieren, diese Aufgabe zu lösen vermag.

Gegenstand der vorliegenden Erfindung ist somit ein Verfahren zur Herstellung von 5-Brom-6-methoxy-1-naphthoesäuremethylester(I) durch Bromierung von 6-Methoxy-1-naphthoesäuremethylester(II) mit Brom, dadurch gekennzeichnet, daß man in Gegenwart eines Lösungsmittels pro Mol 6-Methoxy-1-naphthoesäuremethylester(II) zwischen 0,5 und 0,6 mol Brom (Br₂) einsetzt und die Bromierung zwischen 0°C und der Siedetemperatur des Reaktionsgemisches in Gegenwart eines Oxidationsmittels durchführt, das Bromwasserstoff zu Brom zu oxidieren vermag. Je nach Ausführung der Bromierung können beispielsweise auch Br₂-Mengen von 0,55 bis 0,52 mol pro Mol 6-Methoxy-1-naphthoesäuremethylester(II) eingesetzt werden. Oxidationsmittel, die unter den gegebenen Reaktionsbedingungen Bromwasserstoff zu Brom rückoxidieren können und die keine unerwünschten Reaktionen mit dem Ausgangsmaterial und dem Produkt eingehen, sind beispielsweise Braunstein, Cer-IV-Salze, Alkalimetallbromate oder Peroxyverbindungen; die Rückoxidation des Bromids kann auch elektrochemisch erfolgen.

Bevorzugt wird bei dem erfindungsgemäßen Verfahren als Oxidationsmittel eine Peroxyverbindung eingesetzt. Unter Peroxyverbindungen sind dabei außer dem Wasserstoffperoxid selbst dessen anorganische und organische Derivate zu verstehen, in denen eines der beiden Wasserstoffatome oder beide durch kovalent gebundene Reste oder durch ionisch gebundene Kationen ersetzt sind. Als ionisch gebundene Kationen kommen vor allem die Kationen der Alkali- und Erdalkalimetalle in Betracht, insbesondere Natrium und Barium, als kovalent gebundene Reste zum Beispiel Trialkylsilylreste, Alkylreste, Acylreste, die sich von aliphatischen oder aromatischen Carbonsäuren ableiten, Sulfonylreste oder Reste von anorganischen Säuren. Beispiele für Peroxyverbindungen, die unter den gegebenen Reaktionsbedingungen erfindungsgemäß eingesetzt werden können, sind Natriumperoxid und Bariumperoxid sowie deren Hydrate, Bistrimethylsilylperoxid, tert.Butylhydroperoxid, Peroxyameisensäure, Peroxyessigsäure, Peroxypropionsäure, Peroxylaurinsäure, Peroxystearinsäure, Peroxytrifluoressigsäure, Peroxybenzoesäure, m-Chlorperoxybenzoesäure und Monoperoxyphthalsäure sowie deren Alkali- und Erdalkalimetallsalze, die sogenannten Alkalimetallperborate und -percarbonate wie Natriumperborat und Natriumpercarbonat, Peroxomonoschwefelsäure, Peroxodischwefelsäure, Natrium-, Kalium- und Ammoniumperoxodisulfat, Kaliumperoxomonosulfat, auch in Form seiner Additionsverbindungen mit Kaliumsulfat und -hydrogensulfat, Peroxomono- und Peroxodiphosphorsäure und ihre Natrium- und Kaliumsalze, oder Peroxosalpetersäure, wobei die Peroxyverbindungen nicht nur in Substanz oder in Form handelsüblicher Zubereitungen in das erfindungsgemäße Verfahren eingesetzt werden können, sondern auch erst vor oder während der Durchführung des erfindungsgemäßen Verfahrens in situ entstehen bzw. hergestellt werden können.

Besonders bevorzugt wird bei dem erfindungsgemäßen Verfahren als Oxidationsmittel Wasserstoffperoxid eingesetzt. Verwandt werden vorteilhafterweise insbesondere handelsübliche wäßrige Wasserstoffperoxidlösungen im Konzentrationsbereich 20 bis 50 Gew.%, wie z.B. die 30%ige, 35%ige oder 40%ige Lösung. Es können aber auch Addukte des Wasserstoffperoxids eingesetzt werden, beispielsweise das Addukt mit Harnstoff oder die Addukte mit Natriumboraten oder Natriumcarbonat. Insbesondere bei der Verwendung von Wasserstoffperoxid selbst entsteht gemäß der folgenden Reaktionsgleichung als einziges Abfallprodukt des erfindungsgemäßen Verfahrens dann nur Wasser.

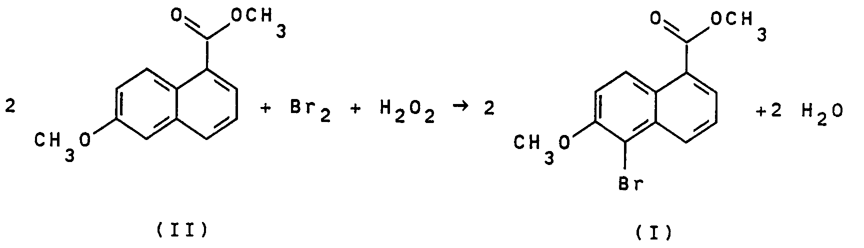

Die Rückoxidation des anfallenden Bromwasserstoffs kann außer mit Einzelsubstanzen auch mit Mischungen von Oxidationsmitteln erfolgen, die Bromwasserstoff zu Brom zu oxidieren vermögen. Ebenso können die als Oxidationsmittel bevorzugten Peroxyverbindungen in Form von Mischungen aus zwei oder mehr Peroxyverbindungen eingesetzt werden bzw. in Form von solchen Mischungen bei der Durchführung des Verfahrens entstehen oder vorliegen. Dies gilt auch für das besonders bevorzugte Wasserstoffperoxid, das auch als Mischung mit einer oder mehreren weiteren Peroxyverbindungen eingesetzt werden oder vorliegen kann.

Die Reihenfolge des Zusammengebens der Reaktionspartner ist variabel. Es kann beispielsweise der Methoxynaphthoesäureester(II) mit dem Oxidationsmittel vorgelegt und das Brom zudosiert werden - im Labormaßstab bei einem 1-mol-Ansatz beispielsweise im Laufe von 30 bis 90 Minuten -, es kann der Ester II vorgelegt und zuerst das Brom, dann das Oxidationsmittel zudosiert werden, es können auch das Brom und das Oxidationsmittel gleichzeitig zum Ester II zudosiert werden. Die benötigte Menge des Oxidationsmittels hängt von der Reaktionsführung ab. Für die Rückoxidation des Bromwasserstoffs zu Brom muß pro Mol Methoxynaphthoesäureester(II) durch das Oxidationsmittel mindestens ein Redox-Äquivalent bereitgestellt werden. Häufig wird ein Überschuß des Oxidationsmittels eingesetzt, um eine vollständige und schnelle Rückoxidation zu erreichen. Wird Wasserstoffperoxid als Oxidationsmittel verwandt, so kann eine äquimolare bis 1,2-fache molare Menge, bezogen auf den 6-Methoxy-1-naphthoesäuremethylester(II), zweckmäßig sein; bevorzugt werden pro Mol II zwischen 0,5 und 0,8 mol, besonders bevorzugt zwischen 0,55 und 0,7 mol Wasserstoffperoxid eingesetzt.

Je nach den Reaktionsbedingungen und dem Oxidationsmittel können verschiedene Lösungsmittel, allein oder in Mischung, für die Durchführung des erfindungsgemäßen Verfahrens geeignet sein, beispielsweise Kohlenwasserstoffe, Ether, Alkohole, Carbonsäuren, Carbonsäureamide oder Wasser. Bevorzugt wird die Umsetzung in einem wassermischbaren Lösungsmittel durchgeführt. In diesem Fall liegt auch dann keine zweite Flüssigkeitsphase vor, wenn das Oxidationsmittel in Form einer wäßrigen Lösung eingesetzt wird, wenn die Löslichkeitsverhältnisse, etwa von Salzen, den Zusatz von Wasser erfordern oder wenn der Zusatz von Hilfsstoffen, etwa von Säuren, notwendig ist. Auch kann in diesem Falle das Waschen des Produkts zum Entfernen des anhaftenden Filtrats und eventueller Salze sofort mit problemlos handhabbarem Wasser erfolgen. Beispiele für geeignete wassermischbare Lösungsmittel sind niedere aliphatische Carbonsäuren, wie Ameisensäure, Essigsäure oder Propionsäure, niedere aliphatische Alkohole, wie Methanol, Ethanol, Propanol oder Isopropanol, Ethylenglykol und seine wassermischbaren Ether, wie Ethylenglykolmono- und -dimethylether, Diethylenglykol und Diethylenglykolmono-und -dimethylether, Tetrahydrofuran, Dioxan, Carbonsäureamide wie Dimethylformamid und N-Methylpyrrolidon. Besonders bevorzugt wird als Lösungsmittel eine niedere aliphatische Carbonsäure oder ein niederer aliphatischer Alkohol verwendet. Ganz besonders bevorzugt ist, daß als Lösungsmittel Essigsäure oder Methanol verwendet wird. Die Lösungsmittelmenge hängt von den Reaktionsbedingungen ab. Das Gewichtsverhältnis 6-Methoxy-1-naphthoesäureester(II) zu Lösungsmittel kann beispielsweise 1 : 2 bis 1 : 20 betragen, je nach Ausführungsform kann z.B. das Verhältnis 1 : 3 bis 1 : 7 und insbesondere das Verhältnis 1 : 4 bis 1 : 6 bevorzugt sein. Dies gilt speziell bei Verwendung von Essigsäure.

Die Durchführung des erfindungsgemäßen Verfahrens kann je nach der Ausführungsart der Reaktion in der Kälte oder in der Wärme erfolgen. Die Umsetzung wird zwischen 0°C und der Siedetemperatur des Reaktionsgemisches, insbesondere zwischen Raumtemperatur und der Siedetemperatur, durchgeführt. Im Laufe der Umsetzung können unterschiedliche Temperaturen eingestellt werden, beispielsweise kann erst beim Zudosieren eine Temperatur zwischen 0°C und 50°C, bevorzugt zwischen Raumtemperatur und 45°C, besonders bevorzugt zwischen 30°C und 40°C, eingehalten werden und anschließend nach beendeter Zugabe zur schnelleren vollständigen Umsetzung von Resten der Ausgangssubstanz II die Temperatur des Reaktionsgemisches auf beispielsweise 70°C oder 90°C oder bis zum Siedepunkt erhöht werden. Die Kristallisation des Produkts beim Abkühlen der heißen Lösung nach vollständiger Umsetzung ist zudem mit einem Reinigungseffekt verbunden.

Die Isolierung des Produkts kann nach üblichen Aufarbeitungsverfahren erfolgen. Ist das Produkt im verwendeten Lösungsmittel nur wenig löslich, so wird die auskristallisierte Substanz nach Abkühlen, beispielsweise auf 30°C oder 20°C oder 10°C oder 0°C, durch Filtrieren oder Zentrifugieren abgetrennt, gewaschen und getrocknet. Bei größerer Löslichkeit des Produkts wird vor dem Abkühlen und dem Abtrennen des Kristallisats beispielsweise ein Teil des Lösungsmittels bei Normaldruck oder im Vakuum abdestilliert.

Nach dem erfindungsgemäßen Verfahren wird der 5-Brom-6-methoxy-1-naphthoesäuremethylester(I) in hoher Ausbeute von ca. 90 % und mit der hohen Reinheit von >98 % (Gehalt durch GC) erhalten, so daß kein zusätzlicher Umkristallisationsschritt für die Weiterverarbeitung notwendig ist. Dies war für den Fachmann nicht vorhersehbar. Aufgrund des Standes der Technik war - wie oben dargelegt - zu erwarten, daß Nebenprodukte aus der Etherspaltung und der Esterverseifung zu Verlusten führen würden, zumal der Reinheitsabfall beim Übergang von 1,2-Dichlorethan als Lösungsmittel, in dem sich der spaltend wirkende Bromwasserstoff nur wenig löst, zur Essigsäure mit ihrem guten Lösevermögen für Bromwasserstoff bekannt war. Umso überraschender ist das gute Ergebnis nach dem erfindungsgemäßen Verfahren erst recht in Anbetracht dessen, daß Wasser zugegen sein kann. Durch den möglichen Verzicht auf chlorierte Kohlenwasserstoffe als Lösungsmittel und durch den mit der starken Reduzierung der Brommenge verbundenen Wegfall der Bromwasserstoffbelastung des Abwassers und der Abluft ergeben sich auch erhebliche arbeitshygienische und ökologische sowie ökonomische Vorteile. Die folgende Übersicht zeigt die Verbesserung, die das Beispiel 1 (Bromierung in Essigsäure) der vorliegenden Erfindung gegenüber dem nächsten Stand der Technik, der in der EP-B 059 596 im dortigen Beispiel 1 g beschriebenen Bromierung des Esters II in Essigsäure, darstellt.

| | erfindungsgemäß | Stand der Technik |
|---|---|---|
| Mol Br₂ pro Mol II | 0,52 | 1,20 |
| theoret. Bromwasserstoffanfall (mol) pro Mol I | 0 | 1 |
| zusätzlicher Umkristallisationsschritt | nein | ja |
| Ausbeute | 92,5 % | 81 % |
| Schmelzpunkt | 126-127,5°C | 119°C |

### BEISPIELE

### 1. Bromierung in Essigsäure

108 g (0,50 mol) 6-Methoxy-1-naphthoesäuremethylester werden in 500 ml Essigsäure gelöst und mit 30 g 35%iger wäßriger Wasserstoffperoxidlösung versetzt. Dann werden bei 35°C über 45 min 42 g (0,26 mol) Brom zugetropft. Anschließend wird auf 90°C aufgeheizt. Nach beendeter Umsetzung läßt man auf 20°C abkühlen, saugt ab und wäscht den Filterkuchen mit Wasser. Durch Trocknen im Vakuum bei 60°C erhält man 136,1 g 5-Brom-6-methoxy-1-naphthoesäuremethylester in Form feiner weißer Nadeln.

| | |
|---|---|
| Ausbeute: | 92,5 % |
| Reingehalt: | >98 % (GC) |
| Schmelzpunkt: | 126 - 127,5°C |

### 2. Bromierung in Methanol

108 g (0,50 mol) 6-Methoxy-1-naphthoesäuremethylester werden in 2500 ml Methanol gelöst und mit 30 g 35%iger Wasserstoffperoxidlösung versetzt. Dann werden bei 35°C über 60 min 42 g (0,26 mol) Brom zugetropft. Anschließend wird auf 70°C aufgeheizt. Nach beendeter Umsetzung engt man im Vakuum auf ein Gesamtvolumen von ca. 500 ml ein, läßt auf 20°C abkühlen, saugt ab und wäscht den Filterkuchen mit Methanol und Wasser. Durch Trocknen im Vakuum bei 60°C erhält man 128,0 g 5-Brom-6-methoxy-1-naphthoesäuremethylester in Form feiner weißer Nadeln.

| | |
|---|---|
| Ausbeute: | 87,1 % |
| Reingehalt: | >98 % (GC) |
| Schmelzpunkt: | 125,5 - 127°C |

## Patentansprüche

1. Verfahren zur Herstellung von 5-Brom-6-methoxy-1-naphthoesäuremethylester(I) durch Bromierung von 6-Methoxy-1-naphthoesäuremethylester(II) mit Brom, dadurch gekennzeichnet, daß pro Mol 6-Methoxy-1-naphthoesäuremethylester(II) zwischen 0,5 und 0,6 mol Brom (Br₂) eingesetzt werden und daß die Bromierung in Gegenwart eines Oxidationsmittels durchgeführt wird, das Bromwasserstoff zu Brom zu oxidieren vermag.

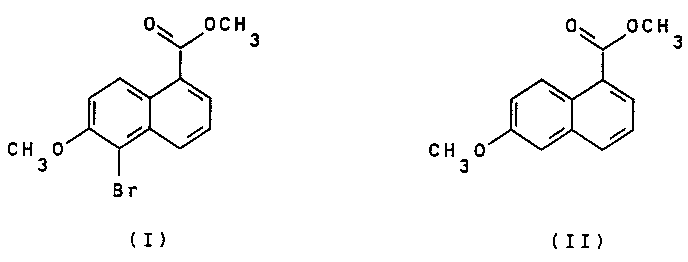

2. Verfahren gemäß Anspruch 1, dadurch gekennzeichnet, daß als Oxidationsmittel eine Peroxyverbindung eingesetzt wird.

3. Verfahren gemäß Anspruch 1 und/oder 2, dadurch gekennzeichnet, daß als Oxidationsmittel Wasserstoffperoxid eingesetzt wird.

4. Verfahren gemäß Anspruch 3, dadurch gekennzeichnet, daß pro Mol 6-Methoxy-1-naphthoesäuremethylester(II) zwischen 0,5 und 0,8 mol Wasserstoffperoxid eingesetzt werden.

5. Verfahren gemäß einem oder mehreren der Ansprüche 1 bis 4, dadurch gekennzeichnet, daß die Umsetzung in einem wassermischbaren Lösungsmittel durchgeführt wird.

6. Verfahren gemäß einem oder mehreren der Ansprüche 1 bis 5, dadurch gekennzeichnet, daß als Lösungsmittel eine niedere aliphatische Carbonsäure oder ein niederer aliphatischer Alkohol verwendet wird.

7. Verfahren gemäß einem oder mehreren der Ansprüche 1 bis 6, dadurch gekennzeichnet, daß als Lösungsmittel Essigsäure verwendet wird.

8. Verfahren gemäß einem oder mehreren der Ansprüche 1 bis 6, dadurch gekennzeichnet, daß als Lösungsmittel Methanol verwendet wird.

9. Verfahren gemäß einem oder mehreren der Ansprüche 1 bis 8, dadurch gekennzeichnet, daß die Umsetzung zwischen 0°C und der Siedetemperatur des Reaktionsgemisches, insbesondere zwischen Raumtemperatur und der Siedetemperatur, durchgeführt wird.

## Claims

1. A process for the preparation of methyl 5-bromo-6-methoxy-1-naphthoate(I) by bromination of methyl 6-methoxy-1-naphthoate(II) with bromine, which comprises employing between 0.5 and 0.6 mol of bromine (Br₂) per mol of methyl 6-methoxy-1-naphthoate(II) and carrying out the bromination in the presence of an oxidizing agent which is capable of oxidizing hydrogen bromide to bromine.

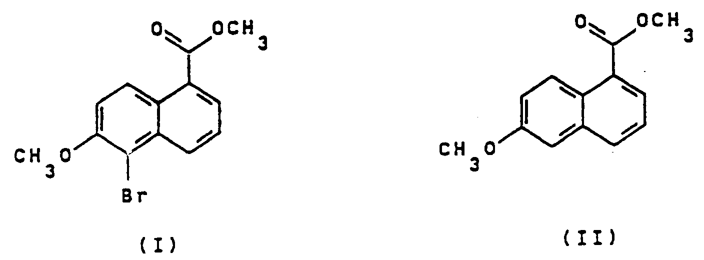

2. The process as claimed in claim 1, wherein a peroxy compound is employed as the oxidizing agent.

3. The process as claimed in claim 1 and/or 2, wherein hydrogen peroxide is employed as the oxidizing agent.

4. The process as claimed in claim 3, wherein between 0.5 and 0.8 mol of hydrogen peroxide is employed per mol of methyl 6-methoxy-1-naphthoate(II).

5. The process as claimed in one or more of claims 1 to 4, wherein the reaction is carried out in a water-miscible solvent.

6. The process as claimed in one or more of claims 1 to 5, wherein a lower aliphatic carboxylic acid or a lower aliphatic alcohol is used as the solvent.

7. The process as claimed in one or more of claims 1 to 6, wherein acetic acid is used as the solvent.

8. The process as claimed in one or more of claims 1 to 6, wherein methanol is used as the solvent.

9. The process as claimed in one or more of claims 1 to 8, wherein the reaction is carried out at between 0°C and the boiling point of the reaction mixture, in particular between room temperature and the boiling point.

## Revendications

1. Procédé de préparation du 5-bromo-6-méthoxy-1-naphtoate de méthyle (I) par bromation du 6-méthoxy-1-naphtoate de méthyle (II) avec du brome, caractérisé en ce que, par mole de 6-méthoxy-1-naphtoate de méthyle (II), on utilise de 0,5 à 0,6 mol de brome (Br₂), et que la bromation est mise en oeuvre en présence d'un oxydant à même d'oxyder le bromure d'oxygène en brome.

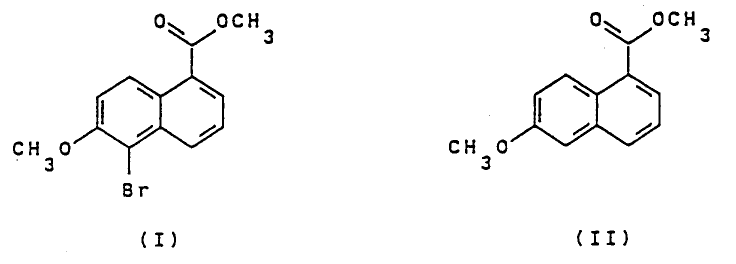

2. Procédé selon la revendication 1, caractérisé en ce qu'on utilise comme oxydant un composé peroxydé.

3. Procédé selon la revendication 1 et/ou 2, caractérisé en ce qu'on utilise comme oxydant du peroxyde d'hydrogène.

4. Procédé selon la revendication 3, caractérisé en ce qu'on utilise par mole de 6-méthoxy-1-naphtoate de méthyle (II) de 0,5 à 0,8 mole de peroxyde d'hydrogène.

5. Procédé selon l'une ou plusieurs des revendications 1 à 4, caractérisé en ce que la réaction est mise en oeuvre dans un solvant miscible à l'eau.

6. Procédé selon l'une ou plusieurs des revendications 1 à 5, caractérisé en ce qu'on utilise comme solvant un acide carboxylique aliphatique inférieur ou un alcool aliphatique inférieur.

7. Procédé selon l'une ou plusieurs des revendications 1 à 6, caractérisé en ce qu'on utilise de l'acide acétique en tant que solvant.

8. Procédé selon l'une ou plusieurs des revendications 1 à 6, caractérisé en ce qu'on utilise du méthanol en tant que solvant.

9. Procédé selon l'une ou plusieurs des revendications 1 à 8, caractérisé en ce que la réaction est mise en oeuvre entre 0°C et la température d'ébullition du mélange réactionnel, en particulier entre la température de réaction et la température d'ébullition.
